# FASCICULE DE BREVET EUROPEEN

(11) **EP 0 448 430 B1**
(45) Date de publication et mention de la délivrance du brevet: **10.01.1996**
(21) Numéro de dépôt: 91400538.4
(22) Date de dépôt: 27.02.1991
(51) Int. Cl.: C12N 9/42

(54) **Composition d'enzymes adaptée à l'hydrolyse des polysaccharides d'un substrat lignocellulosique, sa préparation et son utilisation**
Für die Polysaccharidhydrolyse aus einem lignozellulosehaltigen Substrat geeignete Enzymzusammensetzung, ihre Herstellung und Verwendung
Enzyme composition adapted to the hydrolysis of polysaccharides of a lignocellulosic substrate, its preparation and its use

(30) Priorité: 19.03.1990 FR 9003599
(43) Date de publication de la demande: 25.09.1991
(73) Titulaire: INSTITUT FRANCAIS DU PETROLE, F-92502 Rueil-Malmaison (FR)
(72) Inventeur: Pourquie, Jacques, F-91120 Palaiseau (FR); Warzywoda, Michel, F-92500 Rueil Malmaison (FR)
(74) Mandataire: Andreeff, François

(56) Documents cités:
- US-A- 3 972 775
- BIOTECHNOLOGY AND BIOENGINEERING, vol. 35, janvier 1990, NEW YORK, US, pages 211-216; ALI MOHAGHEGHI et al.
- BIOTECHNOLOGY AND BIOENGINEERING, vol. 17, no. 3, 1975, NEW YORK, US, pages 361-374; N. PETERSEN et al.
- AGRICULTURAL AND BIOLOGICAL CHEMISTRY, vol. 49, no. 6, 1985, TOKYO, JP, pages 1869-1871; Y. MORIKAWA et al.

## Description

La présente invention concerne un procédé pour produire une composition d'enzymes adaptée à l'hydrolyse des polysaccharides d'un substrat lignocellulosique, la composition obtenue et son utilisation

Ces enzymes seront désignés par commodité, cellulases ou composition de cellulases.

Cette composition est appliquée notamment à un procédé d'hydrolyse d'un substrat lignocellulosique prétraité tel que du bois de feuillu, de la paille de céréales et des tiges de maïs.

Dans la perspective d'une production de carburant de substitution à partir de biomasse lignocellulosique par la voie biotechnologique, la production d'enzymes cellulolytiques et hémicellulolytiques demeure une étape critique de tout le procédé pour des raisons aussi bien techniques qu'économiques. Concernant les nombreux microorganiques connus pour excréter des cellulases, le champignon ascomycète *Trichoderma reseei* est reconnu comme l'un des plus susceptibles de donner lieu à une industrialisation de cette production d'enzymes. Mandels (Annual Reports on fermentation processes vol. 5, G. Tsao Editeur, Academic Press NY 1981, p. 35-78) et Morikawa et al. (Agricultural and Biological Chemistry, vol 49, No. 6, 1985, Tokyo, pages 1869-1871) ont montré que les plus fortes productions d'enzymes sont obtenues en fournissant à ce champignon des celluloses purifiées comme principale source de carbone et d'énergie pour sa croissance et sa production de protéines extracellulaires, la production de cellulases étant proportionnelle à la quantité de cellulose apportée au milieu de culture.

L'art antérieur (US-A-3972 775), Biotechnology and Bio-engineering, vol 35, janvier 1990, pages 211-216 et vol. 17, No. 3, 1975, pages 361-374) décrit de plus des substrats pour ces souches, comportant l'utilisation de cellulose seule ou en mélange avec du xylose pour la production d'enzymes cellulolytiques.

L'ensemble de ces documents enseigne donc que le milieu de fermentation doit contenir de la cellulose ou un matériau solide en contenant et qu'il est possible d'en substituer une partie par du xylose pur, sans dépasser cependant une certaine limite au delà de laquelle la productivité est affectée voire stoppée.

L'amélioration des souches sauvages par des techniques classiques de mutation-sélection ont conduit à l'obtention de souches de *Trichoderma reesei* hyperproductrices comme MCG77 (US 4275163) MCG 80 (US 4472504) ou CL847 (Biotechnol. Lett. 1983, 514-243-246) mais leurs productions de cellulases les plus importantes sont aussi reliées à la présence de cellulose purifiée comme substrat carboné principal des milieux de culture. Malheureusement ces milieux de culture ne peuvent pas être extrapolés à l'échelle industrielle en raison, d'une part du coût élevé de la cellulose purifiée et, d'autre part des importantes dépenses d'énergie à consommer pour l'agitation de milieux à fortes concentrations en cellulose (70 à 80 g/l) et donc à fortes viscosités.

C'est pourquoi on observe actuellement une tendance à l'utilisation du lactose comme principal substrat carboné pour cette fermentation. En fait, le lactose est un bon inducteur de la biosynthèse des cellulases et son utilisation dans le cadre d'une production d'enzymes alimentée en fed batch donne des résultats intéressants.

Cependant cette utilisation du lactose souffre de plusieurs inconvénients : le lactose n'est pas facilement disponible dans tous les pays ; il n'est pas nécessairement produit sur le site de production des enzymes. Par ailleurs, le lactose présente une faible solubilité dans l'eau, ce qui implique par comparaison avec d'autres sucres, la manipulation de plus grands volumes de solutions plus diluées et donc des investissements plus importants et des risques plus élevés de contamination.

Il apparaît d'autre part que les cellulases produites à partir de lactose ne sont pas adaptées à l'hydrolyse de n'importe quelle biomasse lignocellulosique. En fait, elle ne montrent une activité intéressante que sur des produits issus de prétraitement à la vapeur effectués en conditions acides.

Ce prétraitement est une opération qui doit être généralement effectuée préalablement à toute saccharification enzymatique de biomasse lignocellulosique. Il a pour objectif la destruction partielle ou totale des différentes barrières anatomiques, physiques ou chimiques, qui font obstacle à l'accessibilité et à l'activité des enzymes sur les polysaccharides cibles.

Bien que différentes technologies de prétraitement aient été proposées, telles que cuissons acides, alcalines ou en présence de solvants organiques, on observe actuellement une tendance à préconiser les prétraitements de type explosion à la vapeur au cours desquels la biomasse lignocellulosique subit une cuisson en présence de vapeur d'eau à haute température (150 à 250°C) pendant un temps de quelques minutes ; cette cuisson est suivie d'une décompression explosive à la pression atmosphérique qui a pour effet d'arrêter les réactions chimiques qui se poursuivaient pendant la cuisson et d'entraîner par l'effet d'explosion une destructuration intense du matériau traité. L'efficacité de l'hydrolyse enzymatique du matériau prétraité dépend de la sévérité des conditions de prétraitement utilisées (température et durée), mais il existe une limite au-delà de laquelle les phénomène de dégradation chimique des sucres au cours de la cuisson deviennent trop importants pour que la valorisation du matériel prétraité soit économiquement intéressante.

Par ailleurs, un des effets du prétraitement à la vapeur est de rendre soluble et d'hydrolyser la fraction hémicellulosique de la biomasse prétraitée, qui peut donc être récupérée après le prétraitement par une simple extraction à l'eau. Dans le cas de prétraitements à la vapeur effectués en conditions acides, obtenues par imprégnation préalable de la biomasse par un acide généralement fort, l'extraction à l'eau permet de récupérer habituellement la totalité de la fraction hémicellulosique sous forme de monomères, principalement de xylose ; le résidu de l'extraction ne contient alors plus que de la cellulose et de la lignine.

Dans le cas de prétraitements à la vapeur effectués sans imprégnation acide préalable, l'extraction à l'eau ne permet de récupérer qu'une partie des hémicelluloses plus ou moins hydrolysées suivant la sévérité des conditions utilisées, l'autre partie restant associée à la cellulose et à la lignine du résidu insoluble.

Ces extraits à l'eau riches en pentoses ou pentosanes peuvent faire l'objet de valorisations spécifiques intéressantes en xylitol ou en furfurol. Malheureusement les marchés de ces produits sont trop étroits pour absorber les productions de pentoses qui résulteraient d'une valorisation à grande échelle des biomasses lignocellulosiques.

Enfin, la saccharification enzymatique des biomasses lignocellulosiques prétraitées, extraites à l'eau ou non, conduit à des solutions de sucres appelées hydrolysats, dont la composition dépend des conditions appliquées antérieurement à ces biomasses mais qui constituent des sources potentielles de substrats carbonés de fermentation.

Les cellulases produites selon le brevet US 4472505 sont connues pour être produites à partir de cellulose pure, à partir de lactose et à partir d'hydrolysats enzymatiques de cellulose pure et sont efficaces pour hydrolyser des celluloses purifiées mais elles ne peuvent être satisfaisantes pour hydrolyser des biomasses lignocellulosiques telles que celles qui résultent des prétraitements à la vapeur effectuées sans imprégnation acide préalable.

Un des objets de l'invention est donc de remédier aux inconvénients décrits ci-dessus.

Plus spécialement, un objet de l'invention est de proposer une nouvelle source de carbone, très facilement disponible et bon marché et plus généralement, un nouveau procédé de fermentation pour la production d'enzymes susceptibles d'hydrolyser des biomasses lignocellulosiques prétraitées.

Un autre objet est de valoriser les extraits de pentoses ou de pentosanes obtenus après prétraitement à la vapeur de substrats lignocellulosiques.

Un autre objet est relatif à la production d'enzymes en quantités suffisantes et présentant les activités d'hydrolyse appropriées à la saccharification enzymatique de biomasses lignocellulosiques prétraitées.

Un autre objet de l'invention concerne l'utilisation de ces enzymes pour la saccharification maximale des biomasses lignocellulosiques prétraitées. On a, en effet constaté de manière inattendue que les activités de saccharification des compositions de cellulases, objets de l'invention, étaient très supérieures à ce que laissaient prévoir les mesures standardisées des activités cellulases (activités papier filtre).

L'invention concerne donc une composition d'enzymes cellulolytiques capable de catalyser l'hydrolyse de biomasses lignocellulosiques diversement prétraitées.

Cette composition d'enzymes cellulolytiques et hémicellulolytiques est obtenue par un procédé comprenant l'inoculation d'une souche de *Trichoderma reesei,* avantageusement améliorée génétiquement dans un milieu de fermentation adéquat et l'incubation de ce milieu ainsi inoculé dans des conditions permettant la croissance de ladite souche et l'excrétion de ses protéines extracellulaires. De manière plus précise, le milieu de croissance consiste essentiellement en une solution aqueuse de sels inorganiques et en une alimentation en sucres hydrosolubles comprenant au moins en partie, un extrait brut de pentoses hydrosolubles provenant du prétraitement d'une biomasse lignocellulosique.

Par l'expression "consiste essentiellement" on entend un milieu de fermentation dans lequel la cellulose ou tout matériau solide en contenant est exclu.

Le procédé de production de cellulases selon l'invention est avantageusement réalisé en fed-batch, selon la technique décrite dans le brevet US 4762788 de la demanderesse.

La composition de cellulases obtenue est caractérisée par les déterminations analytiques usuelles effectuées sur les échantillons de culture débarrassés de la biomasse mycélienne : les protéines solubles sont mesurées selon la méthode de Lowry et al. (Lowry, O.H., Rosebrough, N.J., Farr, A.L. and Randall, R.J. (1951). J. Biol. Chem., 193, 265-275) ; l'activité cellulase dite papier filtre est mesurée selon Montenecourt et al. (Montenecourt, B.S., Eveleigh, D.E., Elmund, G.K. and Parcells, J. (1978). Biotechnol. Bioeng., XX, 297-300) ; l'activité cellobiase est déterminée par mesure du glucose libéré en 30 mn à partir d'une solution 10 mM en cellobiose.

Par extrait brut de pentoses, on entend un extrait de pentoses non purifiés susceptibles de contenir d'autres sucres comme le glucose ainsi que d'autres composés extractibles autres que les sucres (terpènes, composés phénoliques par exemple).

Cet extrait brut pourrait être purifié pour être utilisé dans le cadre de la présente invention.

Les extraits de pentoses utilisés pour l'invention peuvent être obtenus à partir de biomasse lignocellulosique par des moyens connus de l'homme de l'art. Le prétaitement du substrat peut être effectué :
* par cuisson acide, par exemple à 120-150°C environ pendant 2 à 6 heures,
* par la technique dite de l'explosion à la vapeur comprenant une étape de cuisson en présence de vapeur d'eau à haute température pendant quelques minutes (1 à 10 mn) suivie d'une étape de décompression explosive à la pression atmosphérique.
   Cette explosion à la vapeur améliore la susceptibilité à l'hydrolyse enzymatique et solubilise au moins en partie les pentosanes.
   La phase de cuisson précédant la décompression explosive peut habituellement être effectuée soit après imprégnation par un acide fort entre 150°C et 250°C pendant une durée de 1 mn à 10 mn, de préférence 180°C à 200°C pendant 2 à 4 mn, soit sans imprégnation acide, généralement entre 150 et 250°C de 1 mn à 10 mn et de préférence 205 à 220°C pendant 3 à 6 mn.

Selon un premier mode d'obtention des sucres, pour l'alimentation en sucres de la souche, les extraits bruts de pentose peuvent être extraits par resuspension dans l'eau du matériau prétraité suivi d'une filtration de cette suspension.

Selon un deuxième mode, la partie restante, constituant le substrat prétraité, dépentosé en partie, peut être hydrolysée enzymatiquement. On s'arrange généralement pour que les conditions de prétraitement et d'extraction soient ajustées de façon à ce que la teneur en pentoses ou pentosanes soit comprise entre 2 et 30 % de la matière sèche et de préférence supérieure à 5 %, par exemple entre 8 et 25 %.

Selon un troisième mode, le substrat une fois prétraité peut être hydrolysé enzymatiquement et c'est l'hydrolysat enzymatique du substrat prétraité qui constitue une source d'alimentation en sucres pour la souche.

L'hydrolyse enzymatique est réalisée par une préparation de cellulases commerciales appropriées et/ou par une composition de cellulases produites selon l'invention dans les conditions générales suivantes : le matériau à hydrolyser est mis en suspension dans l'eau à raison de 6 à 20 % de matière sèche, de préférence 8 à 12 %, le pH de cette suspension est ajusté entre les valeurs de pH 4 et pH 5,5 de préférence à pH compris entre 4,4 et 4,8, la température contrôlée a des valeurs comprises entre 40 et 60°C de préférence 45 à 50°C ; la réaction d'hydrolyse est habituellement démarrée par addition à la suspension d'une quantité de cellulases ci-dessus correspondant en général à un dosage de 5 à 20 unités papier filtre par gramme de substrat prétraité à hydrolyser, de préférence 10 unités par gramme ; la réaction se poursuit généralement pendant une durée de 15 à 30 heures, de préférence 20 à 24 heures ; le résidu insoluble de lignine est ensuite séparé de l'hydrolysat par filtration ou centrifugation.
On opère de préférence avec les cellulases selon l'invention.

Les enzymes produites selon l'invention sont généralement utilisées dans des procédés d'hydrolyse enzymatique de biomasses lignocellulosiques prétraitées, qui se déroulent habituellement de la manière décrite ci-dessus ; dans ce cas, il n'existe en général pas de limite inférieure à la teneur en pentoses ou pentosanes du produit à hydrolyser. On a constaté que cette application conduisait de manière surprenante à une quantité de sucres produits par ces enzymes, supérieure à ce que l'on pourrait attendre compte tenu de leurs activités papier filtre.

On a également constaté que les cellulases selon l'invention montraient leur supériorité surprenante en présence de substrats non prétraités par voie acide. On évite de ce fait une imprégnation en milieu acide, chaud et on minimise les phénomènes de corrosion.

Les souches avantageusement utilisées sont par exemple les souches hyperproductrices génétiquement améliorées telles que MCG 77 décrite par Gallo dans le brevet US 4275163 ; MCG 80 décrite par Allen, A.L. et Andreotti, R.E., Biotechnology and Bioengineering symposium n° 12, pages 451-459 (1982) ; RUT C 30 décrite par Montenecourt, B.S. et Eveleigh, D.E., Applied Environmental Microbiology, volume 34, page 777 (1977) et CL 847 décrite par Warzywoda, M., et al. Biotechnology letter, volume 5, page 243 (avril 1983).

On a obtenu d'excellents résultats avec *Trichoderma reesei* CL 847 et RUT C 30.

Les matières premières utilisées peuvent être choisies parmi les pailles de céréales, les tiges et rafles de maïs, les plaquettes ou déchets de bois de feuillu ou tout autre source de matériau lignocellulosique présentant généralement au moins 10 % en poids de pentoses ou pentosanes potentiels, par exemple de 15 à 35 % en poids.

L'invention sera mieux comprise au vu des exemples non limitatifs ci-dessous.

### EXEMPLE 1 (comparatif)

On prépare une solution aqueuse de sels minéraux dénommée C1, de composition suivante : 3,32 g/l de potasse (KOH), 4 ml/l d'acide phosphorique à 85 %, 5,6 g/l de sulfate d'ammonium (NH₄)₂SO₄, 1,2 g/l de sulfate de magnésium (MgSO₄, 7 H₂O), 1,2 g/l de chlorure de calcium (CaCl₂), 6,4 mg/l de sulfate de manganèse (MnSO₄), 5,6 mg/l de sulfate de zinc (ZnSO₄, 7 H₂O), 8 mg/l de chlorure de cobalt (CoCl₂), 20 mg/l de sulfate de fer (FeSO₄, 7 H₂).

On prépare une solution aqueuse stérile dénommée C2 comportant pour 1 litre, 250 g de lactose. Un fermenteur contenant 1 litre de solution C1, 0,8 litre d'eau, 2 g/l d'extrait de levure, 2 ml d'antimousse, 2 ml de Tween 80 et 7,5 g de lactose (environ 4,16 g/l) est stérilisé par autoclave pendant 20 minutes à 120°C.

Après refoidissement, la température du milieu est ajustée à 27°C, le pH du milieu à la valeur de pH 5. Le fermenteur est alors ensemencé par 0,2 litre d'une préculture liquide de la souche de *Trichoderma reesei* RUT C 30 ATCC 56765. La température est régulée à 27°C pendant les 24 premières heures de la culture puis à 25°C jusqu'à la fin de la culture. Le pH est régulé à la valeur de pH 5 pendant toute la durée de la culture par addition d'une solution d'ammoniaque ; la concentration en oxygène dissous dans la culture est maintenue au-dessus de 15 % en poids de la concentration saturante par ajustements de la vitesse d'agitation et du taux d'aération.

Des dosages de lactose sont effectués au cours du temps sur des échantillons de culture. Après 10 heures, la concentration en lactose dans le moût de fermentation est de 0,8 g/l. La culture est alors alimentée stérilement de façon continue par la solution C2 ; la vitesse d'alimentation est modulée de façon à ce que la concentration en sucres ne dépasse pas 2 g/l pendant toute la durée de la culture. La vitesse moyenne d'alimentation en lactose est de 1,9 gramme par heure jusqu'à la centième heure, et de 2,25 grammes par heure jusqu'à la fin de la culture. Après 145 heures, 1 litre de la solution C2 a été injectée dans la culture, l'alimentation est alors interrompue et le moût de fermentation est récolté.

Le moût de fermentation est clarifié par centrifugation. Le clarifiat, dénommé E1, constitue la préparation enzymatique. Les déterminations analytiques sur cette préparation E1 donnent les résultats présentés dans le tableau I.

Le potentiel de saccharification de la préparation enzymatique obtenue a été évalué de la façon suivante : deux substrats lignocellulosiques dénommés P_{A} et P_{N} ont été préparés à partir de paille de blé. Pour le substrat P_{A}, la paille est trempée pendant 16 heures dans une solutions 0,08 N en acide sulfurique puis égouttée ; le produit humide subit ensuite une cuisson de 3 minutes sous 18 bar de pression de vapeur d'eau ; cette cuisson est arrêtée soudainement par décompression explosive à l'atmosphère ; le produit obtenu est lavé à l'eau jusqu'à neutralité du pH des eaux de lavage, récupéré par filtration et conservé à basse température. La teneur en hémicelluloses résiduelles du produit P_{A} est inférieure à 3 % de sa matière sèche totale. Le produit P_{N} est préparé suivant un protocole analogue, à l'exception de la phase de trempage acide qui est omise. La teneur en hémicelluloses résiduelles du produit P_{N} est de 23 % de la matière sèche totale.

Les essais de saccharification sont effectués en incubant à 50°C dans un incubateur agité, des fioles d'erlenmeyer de 250 ml, remplies par 100 g de masse réactionnelle de composition suivante : 10 g (exprimés en matière sèche) de substrat P_{A} ou P_{N} à hydrolyser, 50 ml de tampon 100 mM acétate de sodium pH 4,8, 200 mg de protéines de la préparation enzymatique étudiée et une quantité d'eau suffisante pour compléter à 100 g. Après 24 heures, l'incubation est arrêtée en plaçant une partie aliquote des hydrolysats dans un bain-marie bouillant pendant 5 minutes ; les échantillons sont ensuite clarifiés par centrifugation, filtrés sur une membrane 0,2 µ et leur contenu en sucre est analysé par chromatographie liquide haute pression.

La préparation enzymatique E1 a permis d'obtenir les résultats de saccharification suivants : (Tableau Ibis).

**TABLEAU Ibis**

| | Substrat P_{A} (g/l) | Substrat P_{N} (g/l) |
|---|---|---|
| Cellobiose | 9,2 | 2,7 |
| Glucose | 26,6 | 17,9 |
| Xylose | 0,9 | 12,9 |
| Arabinose | 0,3 | 1,1 |
| Total g/l | 37 | 34,6 |

### EXEMPLE 2 (comparatif)

Dans cet exemple, le lactose utilisé dans l'exemple 1 pour la préparation de la solution C2 est remplacé poids pour poids par du xylose commercial pur. Le fermenteur est ensemencé par 200 ml d'une préculture de la souche RUT C 30 de *Trichoderma reesei.* La culture est conduite dans les mêmes conditions que pour l'exemple 1. Après 145 h de culture, la préparation enzymatique E2 est obtenue. Les déterminations analytiques, effectuées sur cette préparation E2, sont présentées dans le tableau I.

L'évaluation du potentiel de saccharification de la préparation E2 donne les résultats suivants (tableau II).

**TABLEAU II**

| | Substrat P_{A} (g/l) | Substrat P_{N} (g/l) |
|---|---|---|
| Cellobiose | 7,9 | 5,6 |
| Glucose | 15,5 | 22,4 |
| Xylose | 0,5 | 15,3 |
| Arabinose | 0,1 | 0,9 |
| Total g/l | 24 | 44,2 |

### EXEMPLE 3

On prépare un extrait brut de pentoses dénommé P1 de la façon suivante : 43 kg de paille de blé sont trempés dans une solution 0,08 N d'acide sulfurique puis égouttés et pressés. Le produit humide obtenu est introduit dans un autoclave équipé d'une vanne de sortie à ouverture instantanée. Dans cet autoclave, le produit subit une cuisson de 3 minutes sous 18 bar de pression de vapeur d'eau à l'issue de laquelle, par détente explosive à l'atmosphère, il est expulsé de l'autoclave dans un cyclone. Le produit solide récupéré au bas du cyclone est extrait deux fois par 180 litres d'eau. Les eaux d'extraction récupérées par filtration sont concentrées par évaporation sous vide jusqu'à obtenir la composition suivante : 315 g/l de xylose, 41 g/L d'arabinose, 50 g/l de glucose.

On prépare une solution aqueuse stérile dénommée C3 comportant 0,616 litre d'extrait P1 et 0,384 litre d'eau. Dans cet exemple, la préparation C2 à base de lactose de l'exemple 1 est remplacée par la solution C3 ; les autres conditions sont égales par ailleurs. Le fermenteur est ensemencé par 200 ml d'une préculture de la souche RUT C 30 de *Trichoderma reesei.* La culture est conduite dans les mêmes conditions que pour l'exemple 1. Après 145 heures de culture, la préparation enzymatique E3 est obtenue. Les déterminations analytiques effectuées sur cette préparation E3 sont présentées dans le tableau I.

L'évaluation du potentiel de saccharification de la préparation E3 donne les résultats suivants (tableau III).

**TABLEAU III**

| | Substrat P_{A} (g/l) | Substrat P_{N} (g/l) |
|---|---|---|
| Cellobiose | 12,06 | 11,7 |
| Glucose | 11,03 | 11,3 |
| Xylose | 0,97 | 16,3 |
| Arabinose | 0,26 | 1,7 |
| Total g/l | 24,34 | 41 |

### EXEMPLE 4

Un hydrolysat pauvre en pentoses, dénommé H1 est préparé de la façon suivante : le résidu solide de la paille traitée et extraite pour obtenir l'extrait P1 de l'exemple 3, est repris dans 250 kg d'eau. Le pH de la suspension est ajusté à pH 4,8 par addition de potasse.700 g de protéines d'une préparation enzymatique produite selon l'exemple 2 sont ajoutés et l'hydrolyse s'effectue à 50°C pendant 20 heures. La suspension obtenue est séparée par filtration en une phase liquide et un résidu solide. La phase liquide est concentrée par évaporation sous vide jusqu'à obtenir la composition suivante : 44,4 g/l de xylose, 296,1 g/l de glucose, 14,3 g/l de cellobiose et 3,2 g/l d'arabinose.

On prépare une solution aqueuse stérile dénommée C4 comportant 0,698 l d'hydrolysat H1 et 302 ml d'eau.

Dans cet exemple, la préparation C2 à base de lactose de l'exemple 1 est remplacée par la solution C4 ; les autres conditions sont égales par ailleurs. Le fermenteur est ensemencé par 200 ml d'une préculture de la souche RUT C 30 de *Trichoderma reesei.* La culture est conduite dans les mêmes conditions que pour l'exemple 1. Après 145 heures de culture, la préparation enzymatique E4 est obtenue. Les déterminations analytiques effectuées sur cette préparation E4 donnent les résultats présentés dans le tableau I.

L'évaluation du potentiel de saccharification de la préparation E4 est présentée dans le tableau IV.

**TABLEAU IV**

| | Substrat P_{A} (g/l) | Substrat P_{N} (g/l) |
|---|---|---|
| Cellobiose | 7,1 | 4,4 |
| Glucose | 24,9 | 15,9 |
| Xylose | 0,9 | 16,5 |
| Arabinose | 0,24 | 1,2 |
| Total g/l | 33,1 | 38 |

### EXEMPLE 5

Un hydrolysat riche en pentoses, dénommé H2 est préparé de la façon suivante : de la paille de blé est prétraitée en conditions acides par explosion à la vapeur suivant un protocole analogue à celui de la préparation du substrat P_{A} de l'exemple 1. 70 kg du produit récupéré après l'explosion sont repris par 180 kg d'eau ; le pH de la suspension est ajusté à pH 4,8 par addition de potasse ; 700 g de protéines d'une préparation enzymatique produite selon l'exemple 7 sont ajoutés et l'hydrolyse s'effectue à 50°C pendant 20 heures. La suspension obtenue est séparée par filtration en une phase liquide et un résidu solide. La phase liquide est concentrée par évaporation sous vide jusqu'à obtenir la composition suivante : 118 g/l de xylose, 42,5 g/l de cellobiose, 197,2 g/L de glucose et 14,3 g/l d'arabinose.

On prépare une solution aqueuse stérile, dénommée C5 comportant 0,672 l d'hydrolysat H2 et 0,328 1 d'eau.

Dans cet exemple, la solution C2 à base de lactose de l'exemple 1 est remplacée par la solution C5, les autres conditions sont égales par ailleurs. Le fermenteur est ensemencé par 200 ml d'une préculture de la souche RUT C 30 de *Trichoderma reesei.* La culture est conduite dans les mêmes conditions que pour l'exemple 1. Après 145 heures de culture, la préparation enzymatique E5 est obtenue. Les déterminations analytiques sur cette préparation E5 sont données dans le tableau I.

L'évaluation du potentiel de saccharification de la préparation E5 est présentée dans le tableau V.

**TABLEAU V**

| | Substrat P_{A} (g/l) | Substrat P_{N} (g/l) |
|---|---|---|
| Cellobiose | 10,2 | 2,11 |
| Glucose | 16,9 | 22,7 |
| Xylose | 0,4 | 15,23 |
| Arabinose | 0,1 | 1,18 |
| Total g/l | 27,7 | 41,22 |

### EXEMPLE 6

La souche CL 847 de *Trichoderma reesei* est cultivée dans des conditions identiques à celles de l'exemple 1. Après 145 heures de culture, la préparation enzymatique E6 est obtenue. Les déterminations analytiques effectuées sur cette préparation E6 sont données dans le tableau I.

L'évaluation du potentiel de saccharification de la préparation E6 est présentée dans le tableau VI.

**TABLEAU VI**

| | Substrat P_{A} (g/l) | Substrat P_{N} (g/l) |
|---|---|---|
| Cellobiose | 8,81 | 1,4 |
| Glucose | 30,39 | 22,4 |
| Xylose | 1 | 14,9 |
| Arabinose | 0,36 | 1,4 |
| Total g/l | 40,56 | 40 |

### EXEMPLE 7

La souche CL 847 de *Trichoderma reesei* est cultivée dans des conditions identiques à celles de l'exemple 2. Après 145 heures de culture sur xylose, la préparation enzymatique E7 est obtenue. Les déterminations analytiques effectuées sur cette préparation E7 sont données dans le tableau I

L'évaluation du potentiel de saccharification de la préparation E7 est présentée dans le tableau VII.

**TABLEAU VII**

| | Substrat P_{A} (g/l) | Substrat P_{N} (g/l) |
|---|---|---|
| Cellobiose | 12,5 | 5 |
| Glucose | 25,7 | 23,4 |
| Xylose | 0,7 | 15,1 |
| Arabinose | 0,3 | 1,1 |
| Total g/l | 39,4 | 44 |

### EXEMPLE 8

La souche CL 847 de *Trichoderma reesei* est cultivée dans des conditions identiques à celles de l'exemple 3. Après 145 heures de culture sur extrait de pentoses, la préparation enzymatique E8 est obtenue. Les déterminations analytiques effectuées sur cette préparation E8 sont présentées dans le tableau I.

L'évaluation du potentiel de saccharification de cette préparation E8, est donnée dans le tableau VIII.

**TABLEAU VIII**

| | Substrat P_{A} (g/l) | Substrat P_{N} (g/l) |
|---|---|---|
| Cellobiose | 12,46 | 8,4 |
| Glucose | 23,71 | 20,5 |
| Xylose | 1,1 | 18,3 |
| Arabinose | 0,3 | 1,2 |
| Total g/l | 37,57 | 48,4 |

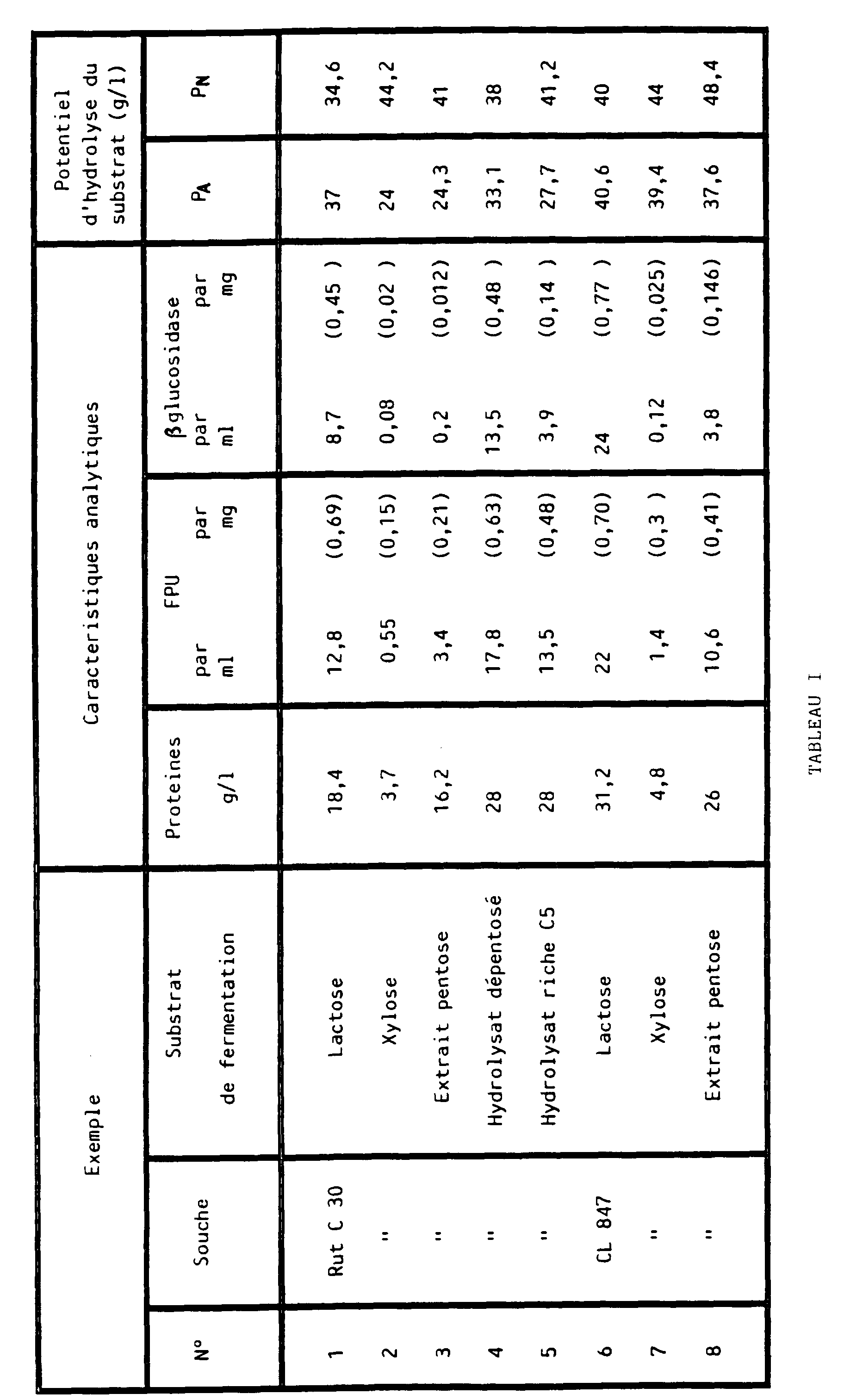

## Revendications

1. Composition d'enzymes cellulolytiques et hémicellulolytiques produite par un procédé où l'on met en présence dans des conditions de fermentation au moins une souche de *Trichoderma reesei* avec un milieu de fermentation carboné caractérisé en ce que ledit milieu de fermentation consiste essentiellement en une solution aqueuse de sels inorganiques et en une alimentation en sucres hydrosolubles comprenant au moins en partie un extrait brut de pentoses hydrosolubles provenant d'un substrat lignocellulosique prétraité avec la condition que la cellulose ou tout matériau solide en contenant est exclu dudit milieu de fermentation.

2. Composition selon la revendication 1 dans laquelle la souche de *Trichoderma reesei* est hyper productrice.

3. Composition selon la revendication 1 ou 2, dans laquelle l'extrait brut de pentoses est contenu dans un hydrolysat enzymatique du substrat prétraité.

4. Composition selon la revendication 1 ou 2, dans lequel l'extrait brut de pentoses est contenu dans un hydrolysat enzymatique du substrat prétraité et en partie dépentosé.

5. Procédé pour la production d'enzymes capables de catalyser l'hydrolyse d'un substrat lignocellulosique prétraité comprenant l'inoculation d'une souche de *Trichoderma reesei* dans un milieu de fermentation carboné adéquat, l'incubation de ce milieu ainsi inoculé dans des conditions permettant la croissance de la souche et la production d'enzymes, le procédé étant caractérisé en ce que le milieu de fermentation consiste essentiellement en une solution aqueuse de sels inorganiques et en une alimentation en sucres hydrosolubles comprenant au moins en partie un extrait brut de pentoses provenant d'un prétraitement d'un substrat lignocellulosique avec la condition que la cellulose ou tout matériau solide en contenant est exclu dudit milieu de fermentation.

6. Procédé selon la revendication 5 dans lequel l'alimentation en sucres provient d'un traitement comprenant une étape de prétraitement du substrat lignocellulosique et une hydrolyse enzymatique du substrat prétraité.

7. Procédé selon la revendication 5 dans lequel l'alimentation en sucres provient d'un traitement comprenant une étape de prétraitement du substrat lignocellulosique, une étape de lavage partiel du substrat prétraité et une étape d'hydrolyse enzymatique du substrat prétraité dépentosé partiellement par le dit lavage partiel.

8. Procédé selon la revendication 5 dans lequel l'alimentation en sucres provient d'un traitement comprenant une étape de prétraitement du substrat lignocellulosique suivi d'une étape d'extraction à l'eau pour récupérer l'extrait de pentoses.

9. Procédé selon l'une des revendications 5 à 8 dans lequel le prétraitement du substrat est réalisé par une cuisson acide, à 120 à 150°C pendant 2 à 6 heures ou par une technique dite de l'explosion à la vapeur comprenant une étape de cuisson du substrat entre 150 et 250°C pendant 1 à 10 mn suivie d'une décompression explosive à la pression atmosphérique.

10. Procédé selon la revendication 9 dans lequel l'étape de cuisson est réalisée en milieu acide fort.

11. Procédé selon la revendication 6 ou 7 dans lequel l'hydrolyse enzymatique du substrat prétraité et l'hydrolyse enzymatique du substrat dépentosé en partie sont effectuées en présence d'une quantité de cellulases commerciales et/ou de la composition de cellulases selon l'une des revendictions 1 à 4, de 5 à 20 unités papier filtre par gramme de substrat prétraité contenant 6 à 20 % de matières sèches durant 15 à 30 heures, à 40-60°C, et à pH compris entre 4 et 5,5.

12. Utilisation d'une composition selon l'une des revendications 1 à 4 ou d'une composition obtenue selon l'une des revendications 5 à 11 dans un procédé d'hydrolyse enzymatique des polysaccharides d'un substrat lignocellulosique prétraité.

13. Utilisation selon la revendication 12 dans laquelle la quantité de la composition correspond à un dosage de 5 à 20 unités papier filtre par gramme de substrat, la température est de 40 à 60°C et la durée de l'hydrolyse est de 15 à 30 heures.

## Patentansprüche

1. Zusammensetzung von zellolytischen und hemizellolytischen Enzymen, die durch ein Verfahren hergestellt wird, bei dem man sie unter Fermentationsbedingungen in Gegenwart von mindestens einen Stamm *Trichoderma reesei* mit einem kohlenstoffhaltigen Medium zusammenbringt, dadurch gekennzeichnet, daß das besagte Fermentationsmedium hauptsächlich aus einer wäßrigen Lösung anorganischer Salze und einem Nährstoff aus wasserlöslichen Zuckern besteht, der mindestens zum Teil aus einem rohen Extrakt wasserlöslicher Pentosen besteht, der aus einem vorbehandelten Substrat aus Lignozellulose besteht, mit der Bedingung, daß die Zellulose oder jedes darin enthaltene feste Material vom Fermentationsmedium ausgeschlossen ist.

2. Zusammensetzung gemäß Anspruch 1, bei der der Stamm von *Trichoderma reesei* hyperproduktiv ist.

3. Zusammensetzung gemäß Anspruch 1 oder 2, bei der der rohe Extrakt von Pentosen in einem enzymatischen Hydrolysat eines vorbehandelten Substrats enthalten ist.

4. Zusammensetzung gemäß Anspruch 1 oder 2, bei der der rohe Extrakt von Pentosen in einem enzymatischen Hydrolysat eines vorbehandelten und zum Teil von Pentosen befreiten Substrats enthalten ist.

5. Verfahren zur Herstellung von Enzymen, welche die Hydrolyse eines vorbehandelten Substrates aus Lignozellulose katalysieren können, das die Animpfung mit einem Stamm von *Trichoderma reesei* in einem geeigneten kohlenstoffhaltigen Fermentationsmedium, die Inkubation des so geimpften Mediums unter Bedingungen, die das Wachstum des Stammes und die Produktion der Enzyme erlauben, umfaßt, wobei das Verfahren dadurch gekennzeichnet ist, daß das Fermentationsmedium im wesentlichen aus einer wäßrigen Lösung anorganischer Salze und einem Nährstoff aus wasserlöslichen Zuckern besteht, der mindestens zum Teil aus einem rohen Extrakt wasserlöslicher Pentosen besteht, der aus einem vorbehandelten Substrat aus Lignozellulose besteht, mit der Bedingung, daß die Zellulose oder jedes darin enthaltene feste Material vom Fermentationsmedium ausgeschlossen ist.

6. Verfahren gemäß Anspruch 5, bei dem der Nährstoff aus Zuckern aus einer Behandlung stammt, die eine Stufe der Vorbehandlung des Lignozellulosesubstrats und eine enzymatische Hydrolyse des vorbehandelten Substrats umfaßt.

7. Verfahren gemäß Anspruch 5, bei dem der Nährstoff aus Zuckern aus einer Behandlung stammt, die eine Stufe der Vorbehandlung des Lignozellulosesubstrats, eine Stufe des teilweisen Waschens des vorbehandelten Substrats und einen Schritt der enzymatischen Hydrolyse des vorbehandelten und durch das besagte teilweise Waschen teilweise von Pentosen befreite Substrat umfaßt.

8. Verfahren gemäß Anspruch 5, bei dem der Nährstoff aus Zuckern aus einer Behandlung stammt, die eine Stufe der Vorbehandlung des Lignozellulosesubstrats, gefolgt von einer Stufe der Extraktion mit Wasser zum Gewinnen des Pentoseextrakts umfaßt.

9. Verfahren gemäß eines der Ansprüche 5 bis 8, bei dem die Vorbehandlung des Substrats durch Kochen mit Säure bei 120 bis 150 °C 2 bis 6 Stunden lang oder durch eine sogenannte Dampfaufschlußtechnik, die eine Stufe des Kochens des Substrats zwischen 150 und 200 °C zwischen 1 bis 10 min, gefolgt von einer plötzlichen Entspannung auf Atmosphärendruck umfaßt, durchgeführt wird.

10. Verfahren gemäß Anspruch 9, bei dem die Stufe des Kochens in stark saurem Medium durchgeführt wird.

11. Verfahren gemäß Anspruch 6 oder 7, bei dem die enzymatische Hydrolyse des vorbehandelten Substrats und die enzymatische Hydrolyse des teilweise von Pentosen befreiten Substrats in Gegenwart einer Menge handelsüblicher Zellulasen und/oder der Zusammensetzung aus Zellulasen gemäß eines der Ansprüche 1 bis 4, 5 bis 20 Einheiten Filterpapier pro Gramm vorbehandelten Substrats, die 6 bis 20 % Feststoffe enthalten und 15 bis 30 Stunden bei 40 bis 60 °C und einem pH zwischen einschließlich 4 und 5,5 durchgeführt werden.

12. Verwendung einer Zusammensetzung gemäß einem der Ansprüche 1 bis 4 oder einer gemäß einem der Ansprüche 5 bis 11 in einem Verfahren der enzymatischen Hydrolyse von Polysacchariden eines vorbehandelten Lignozellulosesubstrats erhaltenen Zusammensetzung.

13. Verwendung gemäß Anspruch 12, bei der die Zusammensetzung einer Dosierung von 5 bis 20 Einheiten Filterpapier pro Gramm Substrat entspricht, die Temperatur 40 bis 60 °C und die Dauer der Hydrolyse 15 bis 30 Stunden betragen.

## Claims

1. A composition of cellulolytic and hemicellulolytic enzymes produced by a process in which at least one strain of Trichoderma reesei is brought into contact with a carbon-containing fermentation medium under fermentation conditions, characterised in that said fermentation medium essentially consists of an aqueous solution of inorganic salts and a hydrosoluble sugar supply at least in part comprising a crude extract of hydrosoluble pentoses derived from a pretreated lignocellulosic substrate, with the condition that cellulose or any solid material containing it is excluded from said fermentation medium.

2. A composition according to claim 1, in which the strain of Trichoderma reesei is hyperproductive.

3. A composition according to claim 1 or claim 2, in which the crude extract of pentoses is contained in an enzymatic hydrolyzate of the pretreated substrate.

4. A composition according to claim 1 or claim 2, in which the crude extract of pentoses is contained in an enzymatic hydrolyzate of the pretreated substrate which is partially depentosed.

5. A process for the production of enzymes capable of catalysing the hydrolysis of a pretreated lignocellulosic substrate, comprising inoculating a strain of Trichoderma reesei into a suitable carbon-containing medium, incubating the inoculated medium under conditions which allow the strain to grow and enzymes to be produced, the process being characterised in that the fermentation medium essentially consists of an aqueous solution of inorganic salts and a hydrosoluble sugar supply at least in part comprising a crude extract of hydrosoluble pentoses derived from a pretreated lignocellulosic substrate, with the condition that cellulose or any solid material containing it is excluded from said fermentation medium.

6. A process according to claim 5, in which the sugar supply derives from a treatment comprising a pretreatment step for the lignocellulosic substrate and enzymatic hydrolysis of the pretreated substrate.

7. A process according to claim 5, in which the sugar supply derives from a treatment comprising a pretreatment step for the lignocellulosic substrate, a partial wash step for the pretreated substrate and an enzymatic hydrolysis step for the pretreated substrate which has been partially depentosed by said partial wash step.

8. A process according to claim 5, in which the sugar supply derives from a treatment comprising a pretreatment step for the lignocellulosic substrate followed by a water extraction step to recover the pentose extract.

9. A process according to any one of claims 5 to 8, in which pretreatment of the substrate is carried out by acid boiling at 120°C to 150°C for 2 to 6 hours or by a vapour explosion technique comprising a boiling step for the substrate at a temperature in the range 150°C to 250°C for 1 to 10 minutes followed by explosive decompression to atmospheric pressure.

10. A process according to claim 9, in which the boiling step is carried out in a strong acid medium.

11. A process according to claim 6 or claim 7, in which enzymatic hydrolysis of the pretreated substrate and enzymatic hydrolysis of the partially depentosed substrate are carried out in the presence of a quantity of commercially available cellulases and/or a cellulase composition in accordance with any one of claims 1 to 4, in a quantity of 5 to 20 paper filter units per gram of pretreated substrate containing 6% to 20% of dry matter for a period of 15 to 30 hours, at 40°C to 60°C, and at a pH in the range 4 to 5.5.

12. Use of a composition according to any one of claims 1 to 4 or a composition obtained in accordance with any one of claims 5 to 11 in a process for the enzymatic hydrolysis of the polysaccharides in a pretreated lignocellulosic substrate.

13. Use according to claim 12, in which the quantity of the composition corresponds to an amount of 5 to 20 paper filter units per gram of substrate, the temperature is 40°C to 60°C and the hydrolysis period is 15 to 30 hours.
